# EUROPEAN PATENT APPLICATION

(11) **EP 1 048 728 A1**
(43) Date of publication of application: **02.11.2000**
(21) Application number: 99201300.3
(22) Date of filing: 26.04.1999
(51) Int. Cl.: C12N 15/30, C12P 1/00, C07K 14/44, C07K 16/20, A61K 39/005, A61K 38/17

(54) **Means and method for the prevention and or treatment of trypanosomosis**

(71) Applicant: Stichting Dienst Landbouwkundig Onderzoek, 6708 PD Wageningen (NL); International Livestock Research Institute ( ILRI), Kabete (KY)
(72) Inventor: Olijhoek, Antonius J. M., 1333 LH Almere-Buiten (NL)
(74) Representative: Ottevangers, Sietse Ulbe

(57) **Abstract**

The invention provides means and methods for obtaining a factor capable of at least in part inducing growth arrest and/or cell death of a trypanosome. Means of the invention include one or more factors that may be purified from culture medium of a culture of trypanosomes, or may be synthesised chemically or produced in a biotechnological process. The invention provides methods for the preparation of medicines for the treatment of Trypanosomosis. The invention further provides vaccines and methods for the preparation of vaccines for the treatment of Trypanosomosis.

## Description

The invention relates to the field of human and veterinary medicine. More particularly the invention relates to the field of the prevention and/or the treatment of diseases caused by parasites, such as Trypanosomosis.

Trypanosomosis is a disease that affects livestock and man in many parts of the world. The disease is for instance widespread in Africa where 300,000 new cases of human sleeping sickness occur each year (TDR: progress 1995-96; chapter 10, African Trypanosomiasis 1997; Vol 13:124-133). A total of 55 million Africans are at risk of contracting the disease. The number of affected livestock is estimated to encompass more than 35 million cattle and an equal number of sheep and goats (WHO Technical Report Series 1986; Vol 739:97-101). Currently, the possibility of combating the disease in livestock and man is very limited. Possible control strategies for trypanosomosis consist of i) disease management through drugs and/or vaccines, ii) vector control, and iii) use of trypano-tolerant cattle. For a variety of reasons none of these approaches has been very successful. Resistance to available drugs has become a severe problem in some countries. In addition, the two drugs that are most effective, melarsoprol and eflornithine, are too expensive for developing countries (TDR: progress 1995-96; Chapter 10, African Trypanosomiasis 1997; Vol 13:124-133). The development of a vaccine has been hampered by the observed extensive antigenic variation in trypanosomes (Barry, JD 1997; Trypanosomiasis and Leishmaniasis: Biology and control, Wellingford, Oxon, UK:CAB International, pages 89-107). Vector control is difficult because of socio-economic factors and although trypano-tolerant cattle don't suffer as much from trypanosomosis, they are more susceptible to other diseases, produce less milk and are less suited for labour (Barret, J. 1997; Trypanosomiasis and Leishmaniasis: Biology and control, Wellingford, Oxon, UK:CAB International, pages 347-359).

Trypanosomes are parasites that are transmitted by an insect vector, the Tsetse fly. The life cycle of the parasite consists of growth of procyclic forms in the fly that differentiate to infective non-dividing metacyclic forms. The metacyclic forms develop to long slender forms in the mammal host and ultimately differentiate to non-dividing Short Stumpy (SS) forms that are pre-adapted for growth in the vector(Vickerman, K 1985: Britsh Medical Bulletin, Vol 41:105-114). The various stages are controlled by complex signalling pathways (Pays, E et al 1997; Trypanosomiasis and Leishmaniasis: Biology and control, Wellingford, Oxon, UK:CAB International, pages 199-225) in which activation of adenylate cyclase enzymes have been shown to play a role (Rolin, S et al 1990, Exp. Parasitol. Vol 71: 350-352; Rolin, S et al 1993, Mol. Biochem. Parasitol. Vol 61:115-125; Rolin, S et al 1996, J. Biol. Chem. Vol 271:10844-10852; van den Abbeele et al 1995, Exp. Parasitol. Vol 81:618-620). Several variant cyclases have been found in *Trypanosoma* (Alexander et al 1990, Mol. Biochem. Parasitol. Vol 43:279-288; Paindavoine, P et al 1992, Mol. Cell. Biol. Vol 12:1218-1225; Ross et al 1991, EMBO J. Vol 10:2047-2053), which may act as specific receptors for the transition of one growth stage to another.

In mammals, the density of parasites in the blood varies in a wave-like fashion, in time (Barry, D et al 1991, Parasitol. Today Vol 7:207-211). The varying density of parasites in the blood has often been exclusively attributed to the immune response of the infected host (McLintock, LM et al 1993, Parasite Immunol. Vol 15:475-480). Several host factors, like for example EGF and transferrin, have been shown to influence the parasite growth rates(Turner et al 1996, Parasitol. Res. Vol. 82:61-66). Parasite growth control in infected animals, is generally attributed the accumulation of (a) factor(s) in the blood, other than EGF and transferrin. Such a mechanism seems to be supported by a simulation model for growth control as proposed by Seed and Black (Seed , JR and Black SJ, 1997, J. Parasitol. Vol 83:656-662). The origin of the postulated control factor(s) has remained elusive. The actual number and the origin of such control factor(s) is not known. While some authors hypothesised a host origin, others favoured a parasite origin. Growth control of parasites in the insect vector has also been under study. One mechanism of control in the insect vector act by means of inducing apoptosis.

In the present invention the concept of factors produced by parasites was investigated to find novel treatment modalities for trypanosomosis. The present invention describes novel means and methods for at least controlling growth, differentiation and/or cell death of trypanosomes, particularly of bloodstream forms of trypanosomes. In one aspect a means of the invention is a factor capable of being produced by a trypanosome in culture. Said factor can also be produced in other ways. Preferably said factor is produced in a comparatively inexpensive fashion. Moreover, a factor of the invention is preferably capable to exert its effect on a range of different trypanosomes.

In one embodiment the invention provides a method for obtaining a factor capable of at least in part inducing growth arrest and/or cell death of a trypanosome comprising harvesting culture medium of a culture of trypanosomes and purifying said factor from said culture medium. Purification of said factor may be performed through any suitable means. Preferably, said purification comprises fractionation of said culture medium. In a preferred embodiment said purification comprises
- freeze drying said culture medium,
- dissolving freeze dried medium with a solution of ammonium bicarbonate (0,1 M),
- subjecting dissolved freeze dried culture medium to ultrafiltration through an Amicon YM2 filter,
- subjecting the filtrate of said ultrafiltration to a gel filtration on a sephadex G10 gelfiltration column,
- eluting said factor from said gelfiltration column with a solution of ammonium bicarbonate (0,1 M) and collecting fractions.
- freeze drying collected fractions suspected of comprising said factor,
- dissolving said freeze dried material with an aqueous solution comprising 0.1% trifluoro acetic acid,
- subjecting dissolved freeze dried material to reverse phase high pressure liquid chromatography and collecting fractions.

Said culture may be any culture of trypanosomes. Preferably said culture is a late log phase culture of trypanosomes.

A factor of the invention may be purified from culture medium of any strain of trypanosome. Preferably said culture of trypanosomes is a culture of *Trypanosoma congolense* and/or *Trypanosoma brucei.*

In one aspect the invention provides a factor obtainable by a method of the invention. Said factor may also be obtained in other ways, for instance through chemical synthesis or biotechnological means. In one embodiment of the invention said factor has an apparent Molecular Weight of less than 2000 Dalton. Preferably said factor has an apparent Molecular Weight of less than 1000 Dalton.

In another embodiment of the invention said factor is capable of at least in part inducing growth arrest and/or cell death of a bloodstream form trypanosome. In a preferred embodiment said factor is capable of at least in part inducing growth arrest and/or cell death of at least two, more preferably at least three different strains of trypanosomes, preferably of *Trypanosoma congolense, Trypanosoma* brucei and *Trypanosoma vivax.*

In one embodiment of the invention said factor is capable of interfering at least in part with the function of an adenosinediphosphate ribosylation factor (ADP-ribosylation factor or ARF), preferably a trypanosome associated ARF.

ARF's belong to a Ras-related family of small GTP-binding proteins. Other members of the family are Rab and Sarl (Exton, JH, 1998, J. Biol. Chem. Vol 273, p 19923; Moss, J and Vaughan, M 1998, J. Biol. Chem. Vol 273, p21431-21434). ARF proteins are involved in the regulation of vesicular membrane trafficking in both endocytotic and exocytotic pathways. ARF proteins seem to act by controlling assembly of transport vesicles. Like all small GTPases, ARF proteins function as molecular switches by cycling between active GTP-bound and inactive GDP-bound forms (Nuoffer, C and Balch, WE 1994, Ann. Rev. Biochem Vol 63:949-990). ARF proteins have a requirement for lipids in order for activation to occur (Franco, M et al 1996. J. Biol. Chem. 271:1573-1578), this in contrast to other members of the family (Randazzo, PA. and Kahn, RA 1995, Methods in Enzymol. 250:394-405). After binding to the membrane, ARF proteins are activated by GTP exchange proteins which themselves seem to be regulated by PIP2 (Chabre et al 1998, Trends Biochem Sci. Vol 23:98-100; Mossessova et al 1998, Cell Vol 92:415-423)). ARF has been shown to bind to coatomers (Gaynor et al 1998, Mol. Cell. Biol. Vol 9:653-670; Lippincott-Schwartz et al 1998, Histochem. Cell Biol. Vol 109:449-462) and Adaptor Proteins 1 and 3 (Ooi et al, 1998, J. Cell. Biol. Vol. 142: 391-402; Boman and Kahn, 1995, Trends Biochem Sci. Vol 20:147-150) which are specific proteins required for vesicle formation. Direct activation of phospholipase D by ARF seems to play a role in this process as well (Kim et al 1998, FEBS Letters, Vol 430:231-235). In the case of endocytosis, GTPase activity of ARF is required for shedding of the vesicular membrane proteins and fusion of the vesicles with the lysosomal membrane (Moss and Vaughan, 1998, J. Biol. Chem. 273:21431-21434). After processing in the lysosome, ARF and receptor proteins are relocated to the cytoplasmic membrane, thereby completing the cycle.

The first steps in endocytosis are still not well understood. In one study ARF have been found to co-localise with transferrin receptors that were to be internalised. Thus, the direct interaction of ARF with the respective receptors (DqSouza-Schorey et al 1998. J. Cell. Biol. Vol 140:603-616) could be a possible first step in the uptake process.

To date, very few ARF genes have been cloned from parasites (Truong et al, 1997, Mol. Biochem. Parasitol. Vol. 84:247-253; Murtagh et al 1992, J. Biol. Chem. Vol 267:9654-9662). In the present invention we present evidence that a cloned and recombinantly expressed trypanosomal ARF protein can interact with at least one factor present in culture medium of cultured trypanosomes.

In one aspect the invention provides a factor capable of at least in part inducing growth arrest and/or cell death of a trypanosome, capable of interacting with a trypanosomal ARF, wherein said interaction contributes at least in part to the observed growth arrest and/or cell death of said trypanosome.

In another aspect the invention provides an antibody with a specificity for a factor of the invention. The invention further provides an antibody with a specificity for a binding site of a factor of the invention on ARF. The invention further provides an antibody with a specificity for an ARF bound to a factor of the invention.

In another aspect the invention provides an anti-idiotypic antibody of an antibody of the invention.

An antibody of the invention may be an antibody or a functional part, derivative and/or analogue thereof. A non-limiting example of a suitable part is a FAB-fragment. A non-limiting example of a suitable derivative is a single chain antibody. A non-limiting example of a suitable analogue is a synthetic antibody selected from a recombinant antibody library. Proteinaceous molecules with similar binding characteristics, in kind not necessarily in amount, as the antibodies of the invention are non-limiting examples of suitable analogues of the antibodies of the invention. Such proteinaceous molecules may be obtained by performing screening assays in which many different proteinaceous molecules are tested for their binding specificities. In a preferred embodiment an antibody of the invention is a monoclonal antibody or a functional part, derivative and/or analogue thereof.

In yet another aspect the invention provides a proteinaceous molecule capable of binding to ARF, preferably a parasite derived ARF, more preferably a trypanosome derived ARF. Preferably said proteinaceous molecule is capable of binding to at least part of a binding site of a factor according to the invention on ARF or a functional part, derivative and/or analogue of said binding site. Preferably said ARF is a Trypanosome ARF.

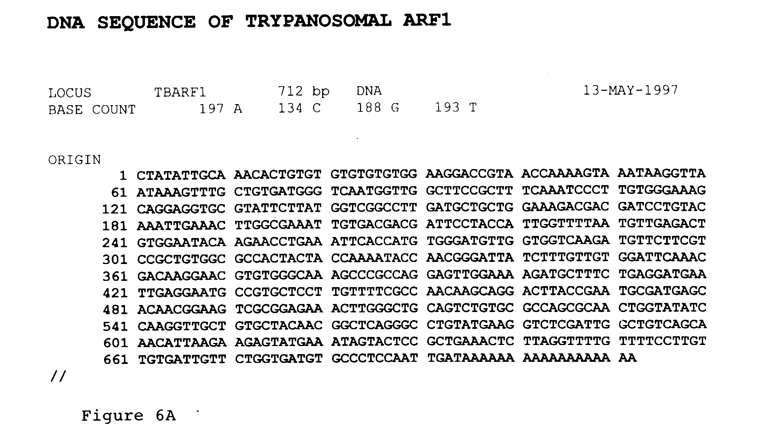

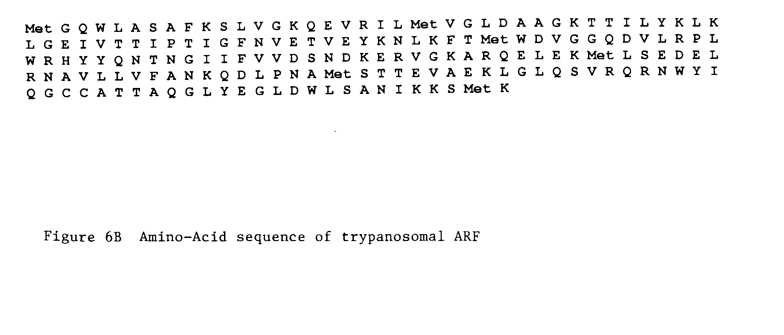

In one aspect the invention provides an isolated and/or recombinant nucleic acid encoding an ARF encoded by a trypanosome. Preferably said trypanosome is a *Trypanosome brucei.* Preferably said nucleic acid comprises a sequence as depicted in figure 6A, or a functional part, derivative and/or analogue thereof. Suitable derivatives can be generated through using codon degeneracy. Alternatively, ARF may be isolated and cloned from other trypanosomes through screening cDNA and/or genomic libraries by screening using said nucleic acid under stringent hybridisation conditions or alternatively expression cDNA libraries of trypanosomes may be screened with one or more antibodies binding specifically to said *Trypanosome brucei* ARF. The invention further provides an ARF or a functional part, derivative and/or analogue thereof encoded by said isolated and/or recombinant nucleic acid. Preferably, said ARF comprises an amino-acid sequence as depicted in figure 6B, or a functional part, derivative and/or analogue thereof.

In yet another aspect the invention provides a method for the treatment of an individual suffering from or at risk of suffering from Trypanosomosis comprising administering to said individual a factor according to the invention, an antibody according to the invention and/or a proteinaceous molecule according to the invention. Preferably, said individual is a human, or a life-stock animal.

In yet another aspect the invention provides a pharmaceutical composition comprising a factor according to the invention, an antibody according to the invention and/or a proteinaceous molecule according to the invention, and a pharmaceutically acceptable carrier.

The invention further provides the use of a factor according to the invention, an antibody according to the invention and/or a proteinaceous molecule according to the invention, as a pharmaceutical.

The invention further provides the use of a factor according to the invention, an antibody according to the invention and/or a proteinaceous molecule according to the invention, for the preparation of medicament for the treatment of Trypanosomosis.

In another aspect the invention provides a vaccine for preventing and/or treating at least in part Trypanosomosis comprising an antibody according to the invention and/or a proteinaceous molecule according to the invention, and a suitable adjuvant. Preferably said antibody and/or said proteinaceous molecule is capable of binding to a trypanosome derived ARF.

### EXAMPLES

### Materials and Methods

### Trypanosomes.

The following strains of Trypanosomes were used : *Trypanosoma congolense* 1180 was isolated in the Serengeti, Tanzania (Geigy R and Kauffman M (1973) Acta Trop. Vol 3, pp 12-23; Nantulya VM et al (1984) Infect. Immun Vol 43, 735-738)

*Trypanosoma brucei* in the present invention is *Trypanosoma brucei brucei* ILTat1.1 (Miller FN and Turner MJ (1981), Parasitology Vol 82, pp 63-80) which is a cloned derivative of EATRO (East African Trypanosomiasis Research Organisation) 795 which was isolated form a cow in Uhembo, Kenya

*Trypanosoma* vivax is a rodent infective stock termed IL1392 which is a derivative of ZARIA Y486 isolated in 1973 from a cow in Nigeria (Leeflang et al (1976) Int. J. Parasitol. Vol 6, pp 413-417).

Trypanosoma congolense bloodstream forms are propagated *in vitro*, axenicaly using a simplified cultivation medium at 34 °C in a humidified atmosphere of 4.5% CO₂ in air (Kaminsky, R., Chuma, F., Wasike, P (1994). Veterinary Parasitology Vol XX, pp XX-XX).

The cultivation medium is a simplified liquid medium introduced by Hurimi, H and Hurimi, K (1991) Parasitology Vol. 102, pp 225-236. The simplified medium does not have "serum plus" but goat serum. In our experience not only young goat serum but also adult serum can support trypanosome growth (Chuma, F. and Kaminsky, R., unpublished data). For culture of *T. vivax* and *T. brucei* goat serum was replaced by foetal bovine serum.

Cultivation medium is an Iscoves modified Dulbecco's Modified Eagles Medium DMEM, Flow, Irvine, Scotland: Cat no. 10-355-22), supplemented with:
- 0.05 mM Bathocuprion disulphonic acid (Sigma, Yabu et al, 1989, Parasitol Res. Vol 76, pp 93-97)
- 1.5 mM L-cysteine (Calbiochem, Duszenko et al, 1985, J. of Exp. Med. Vol 162, 1256-1263, Hurimi, H and Hurimi, K (1991) Parasitology Vol. 102, pp 225-236).
- 0.5 mM Hypoxanthine (Calbiochem)
- 0.12 mM 2-mercaptoethanol (Balz et al, 1985, EMBO J. Vol 4, pp 1273-1277).
- 1 mM Sodium pyruvate (Sigma)
- 0.16 mM Thymidine (Calbiochem)
- 2 mM L-Glutamine (Gibco)
- 20% Caprine serum, previously heat inactivated at 56 °C for 30 minutes.

The supplements are added whereupon the medium is mixed and filtered through 0.22 µM filters (Millipore). The resulting cultivation medium is subsequently dispensed into 50 ml aliqouts and stored at 4 °C until use. Unused cultivation medium is discarded after three weeks storage at 4 °C.

### Cultivation of Trypanosome congolense

*In vitro* cultivation of *Trypanosoma congolense* bloodstream cultures is initiated either by :
a) metacyclics derived from *in vitro* cultures of epimastigotes. The metacyclics are purified away from the epimastigotes by DE 52 column chromatography or,
b) parasites obtained from the blood of mice infected with metacyclics derived from in vitro cultures, then on rising parasitaemia, bloodstream form parasites are obtained and in vitro cultures set up (Hurimi and Hurimi, 1989 J. Parasitol. Vol 75, pp 985-989).

Ad a) Metacyclic forms for initiating bloodstream forms are obtained by the procedure described by Gray et al, 1981, Parasitology Vol 82, pp 81-95), but the insect form cultures are initiated from mouse derived bloodstream forms. Essentially, metacyclic forms are harvested, concentrated and finally adjusted to 1 x 10⁶/ml in cultivation medium. These are seeded as follows:
a) In 24 well tissue culture plates (Costar, Cambridge, MA, USA) or,
b) In T25 tissue culture flasks (25 cm² Costar, Cambridge, MA, USA).

For a 24 well plate add 1 ml of the cultivation medium. Add 5 ml of the cultivation medium into each T25 tissue culture flask. Cultures are incubated at 34 °C in the presence of 4.5% CO₂. Cultures of trypanosomes may be supplemented with Bovine Aorta cell feeder layers.

Ad b) Direct initiation of mouse derived bloodstream form cultures.

Harvest of mouse trypanosomes: Clip the top of a mouse tail and collect aseptically blood (approximately 100 µl) in an eppendorf tube and dilute with medium.

For a 24 well plate: Add 0.5 ml of cultivation medium in a well of a 24 well plate. Using a 10 µl eppendorf pipette take 10 µl of the mouse tail blood and place it a corner of the well.

For a T25 flask: Add 2.5 ml of cultivation medium to the flask and add 100 µl of the mouse tail blood, in the middle near the mouth of the flask.

Two hours after initiation of the culture the medium is collected and inserted in a new well or flask.

Change the culture medium after every 24 to 48 hours by replacing at most half the volume with fresh cultivation medium.

### Preparation of stock solutions.

Bathocuproin disulfonic acid: Dissolve 28.2 mg bathocuproin disulfonic acid in 10 ml Iscoves medium. Store aliquots of approximately 1 ml each at -20 °C).

Hypoxanthine : Dissolve 68.1 mg hypoxanthine in 10 ml of Iscoves medium. Store aliquots of approximately 1 ml each at -20 °C).

Thymidine : Dissolve 38.8 mg thymidine in 10 ml of Iscoves medium. Store aliquots of approximately 1 ml each at -20 °C).

Sodium pyruvate : Dissolve 220 mg sodium pyruvate in 10 ml of triple distilled water. Store aliquots of approximately 1 ml each at -20 °C).

For the preparation of medium use 1 ml of each of the stock solutions above mentioned for every 100 ml of medium.

L-Cystein: immediately prior to preparation of cultivation medium weigh 26.3 mg L-cystein and add it to 100 ml of medium.

2-Mercaptoethanol: Add 14 µl of analysis grade Mercaptoethanol into 10 ml of water. Use 600 µl of this dilution for every 100 ml of medium.

### Purification of biologically active fractions from conditioned growth medium.

Trypanosomes were cultured to late log phase in 2 litter of medium. The medium was removed by centrifugation (3000 RPM, 15 minutes) and replaced with fresh medium without serum. The cultures were incubated for another 4 hours whereupon the conditioned medium was harvested by pelleting the trypanosomes and filtering the supernatant through Millipore 0.45 micrometer pore size filters. The conditioned medium was freeze dried and redissolved in 40 ml of 0.1 M Ammonium Bicarbonate.

Concentrated conditioned medium was subjected to ultrafiltration through Amicon YM2 filters and the 2 ml samples of the filtrate were applied to 200 ml Sephadex G10 gel filtration column. Alternatively the concentrate was directly applied to the column. Fractions were eluted with 0.1 Molar Ammonium Bicarbonate at a flow rate of 1.5 ml / min. and the absorption at 220 nm was recorded. Collected fractions (7.5 ml) were lyophilised and redissolved in 1.0 ml of 0.1 % TFA / water. For RPLC 0.5 ml fractions were applied to a PepS column (Pharmacia ) at a flow rate of 0.5 ml / min. The collected RPLC fractions (0.5 ml) were stored at - 30 °C. These fractions contained 100 x the activity of the original culture medium.

### Biological activity.

The collected fractions from Gel Filtration and RPLC were tested for activity by adding 10 ul of concentrated fractions to 0.5 ml cultures in 24 well plates seeded at 10⁵ parasites / ml of growth medium. Cultures were followed by microscopic observation. Growth curves were obtained by counting cell numbers twice daily for a period of 4 days.

### TUNEL Assay and Rhodamine staining.

Cells on slides made by cytospinning of 200 microlitre culture medium of cultures 48 hours after addition of concentrated fraction F2 (see below) were labelled using the Fluorescein In Situ Cell Death Detection Kit (Cat. Nr 1684795; Boehringer-Mannheim, Germany). The slides were pretreated in a microwave oven (setting 8, 30 seconds), alternatively slides were pretreated by immersion in acetone at -20 °C for 30 minutes. Labelling was done according to the manufacturers instructions. Rhodamine staining for total DNA was done on the same slides and micrographs were taken using a Nikon Fluorescence microscope.

### Staining procedures.

Aliquots of 10 ul of concentrated RPLC fractions were spotted on paper and sprayed with Ninhydrin reagent for the detection of peptides. The same amount was spotted on silica coated strips and treated with Bial's Orcinol reagent (Milton Roy Company, PA 16801, USA) or with o-anisidine reagent (600 mg of o-anisidine dissolved in 5 ml acetone, 94 ml of methanol and 0.6 ml of orthophosphoric acid added) for the detection of sugar. After dipping in the respective reagents TLC plates were developed at 120 °C for 10 minutes.

Lipids were detected by exposing silica coated strips to Iodine vapour in a closed tank.

Cultures were prepared for microscopy the following way. 0.5 ml of cultures grown for 48 hours with or without medium extracted factors were concentrated onto glass slides by cytospin centrifugation at 1500 rpm for 10 minutes (Shandon Southern Cytospin centrifuge). The slides were dried and stained with Giemsa. Photographs of these slides were taken using a microscope mounted with a camera.

### Reverse phase high pressure liquid chromatography (RPLC).

For RPLC we used a Pharmacia system consisting of UV detector VWM 2141, gradient pump 2249 and a Helifrac fraction collector. The column was a SuperPac Pep-S 4x250 mm column (5 um particle size). Samples were loaded using a 500 ul loop. The solvent system consisted of solvent A: 0.1 % TFA in water, solvent B: 0.1 % TFA in 90 % acetonitrile. All reagents were HPLC grade.

### Thin Layer Chromatography

Thin Layer Chromatography was done using precoated polyester backed silica plates (Whatman, PE SILG /UV). Samples were spotted in a thin line at 2 cm from the bottom of the plates. Separation was performed in a sealed tank using n-propanol/water 70/30 as the solvent. The usual running time was 6 hours at room temperature.

### Affinity chromatography.

Recombinant of E. coli strains expressing ARF or NH were grown in 1 liter culture volumes by inoculation of a single colony of the recombinant strain in 50 ml of Tryptic Soya Broth standard medium (TSB medium) overnight under shaking in a 37 °C incubator. The following day 2 ml of this culture was inoculated in 1 liter of TSB and grown to late log phase (approximately 6 hours). Expression of the protein was induced by addition of IPTG (2.5 micrograms/ml) and incubation was continued for 1,5 hours. Cells were centrifuged (Beckman centrifuge; 8000 rpm, 30 minutes) and the pellets were redisolved in 20 ml of sonification buffer (200 mM Na₂HPO₄ pH 8.0, 300 mM NaCl). The celsuspensions were subsequently sonicated for 25 minutes using a Branson sonicator, a microtip, maximum output setting and 50% cycle. After sonification the suspension was centrifuged in an eppendorf centrifuge at 13000 x g for 30 minutes. The combined pellets were discarded and the clear lysate was filtered through a millipore 0.45 µM filter and stored at -80 °C. Columns were prepared by absorbing 10 ml of these lysates on 0.5 ml Ni resin (Novagen). The columns were washed according to the manufacturers instructions. Two ml of 50 x concentrated supernatant of T. congolense was incubated with the ARF-bound resin material for 3 hours at room temperature. Not bound supernatant material was removed and the columns were washed using the standard buffers supplied. For elution we used the same buffer after adjusting the pH to 4.0.

### Lectin column chromatography.

Lectin columns were prepared using 1 ml of RCA-1 coupled resin (Vector Labs, CA, USA). The protein concentration in the resin was 4.0 mg/ml settled gel. Two ml of 50 x concentrated supernatant of T. congolense was incubated with the resin material for 3 hours at room temperature. Not bound material was removed and the columns were washed using the affinity column buffers. Elution was done using the pH 4.0 eluting buffer with 200mM lactose added.

### Results

### Biological effects of separated fractions on growing trypanosomes.

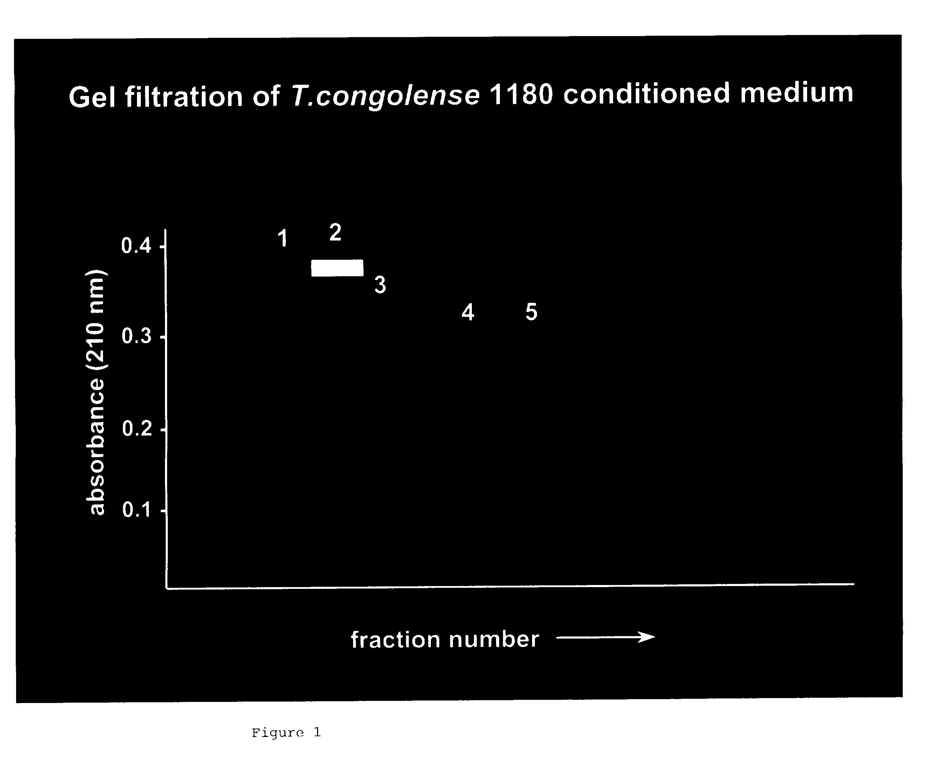

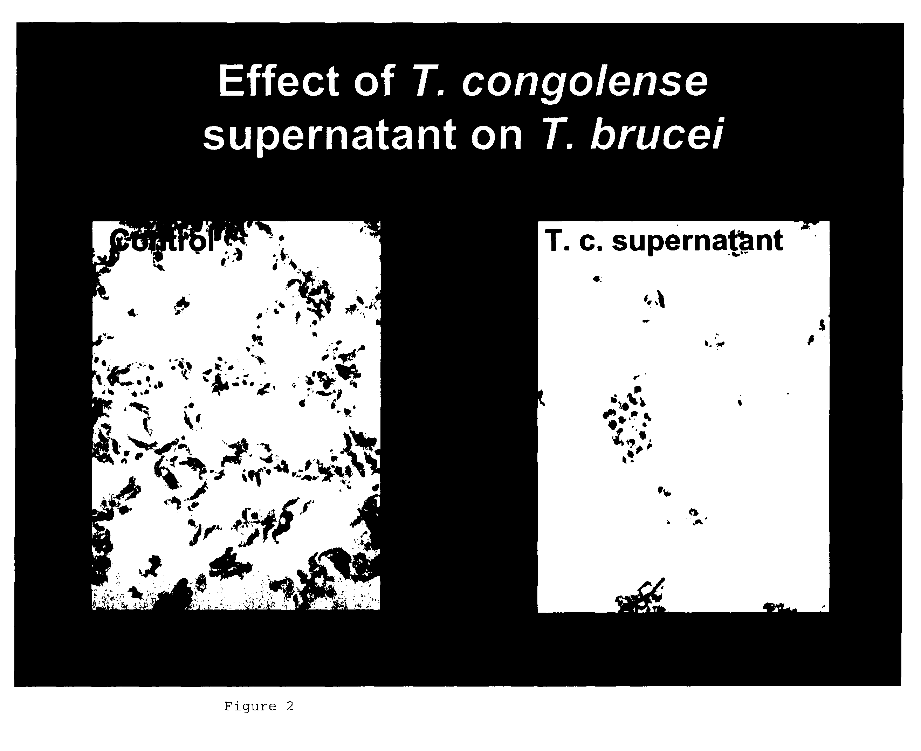

Fig. 1 shows the typical separation profile obtained by gel filtration of conditioned medium. Of these fractions from conditioned medium of *T. congolense*, the fraction marked F2. had the highest activity on *T. congolense, T. b. brucei* and on *T. vivax*. The effect of this fraction on a culture of T. brucei is shown in Fig. 2. Similar profiles were obtained using conditioned medium after growth of the other parasites.

Cell growth was markedly affected by the addition of F2 in all three species of trypanosomes.

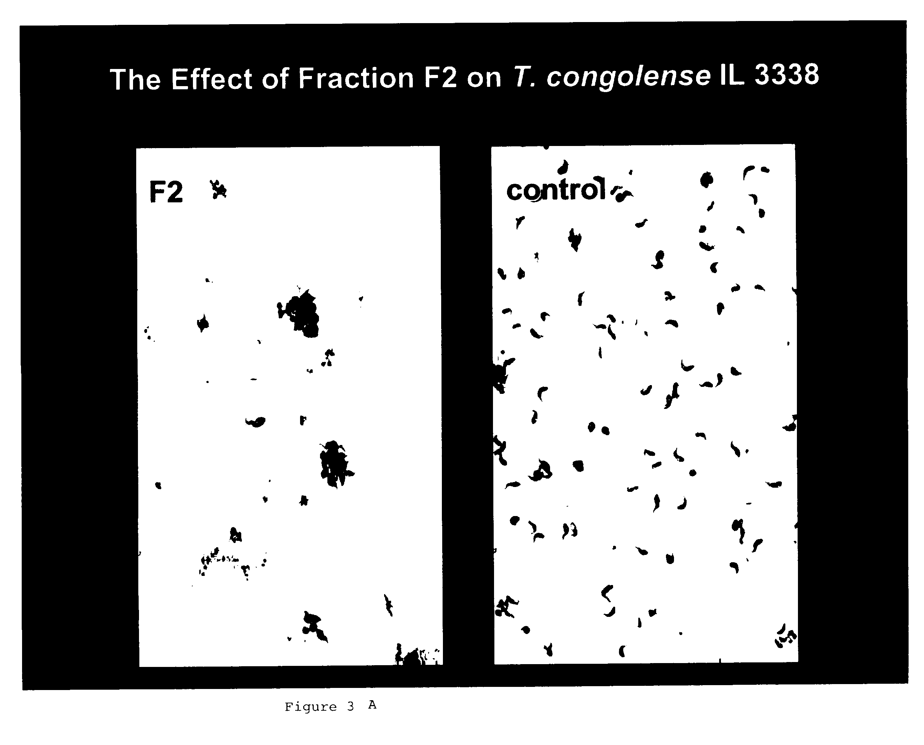

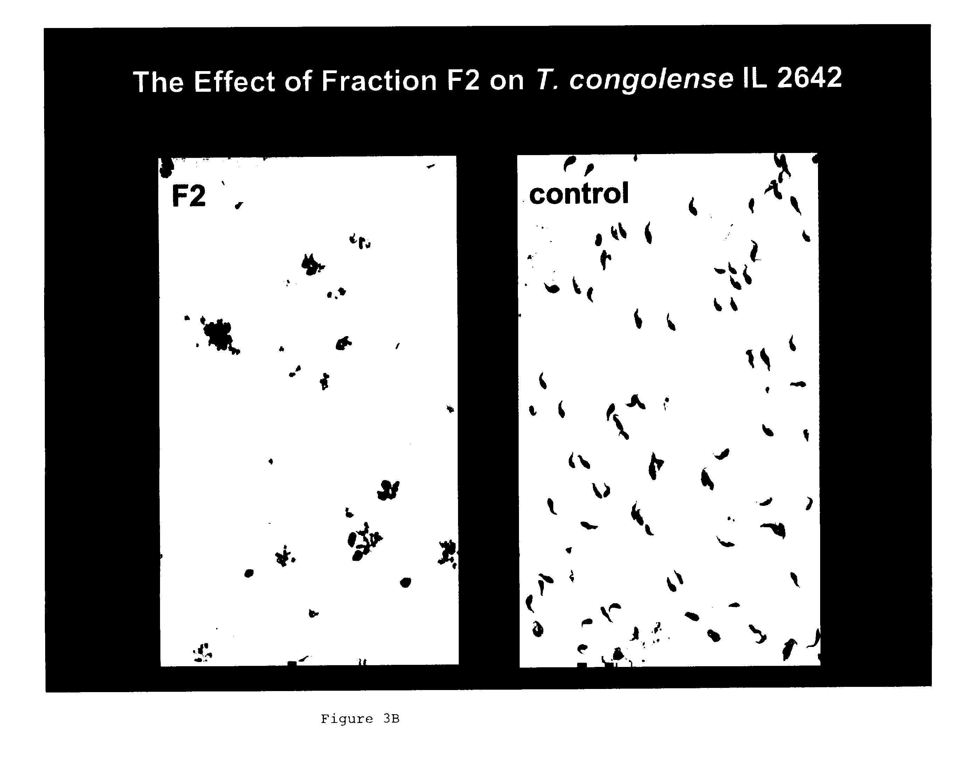

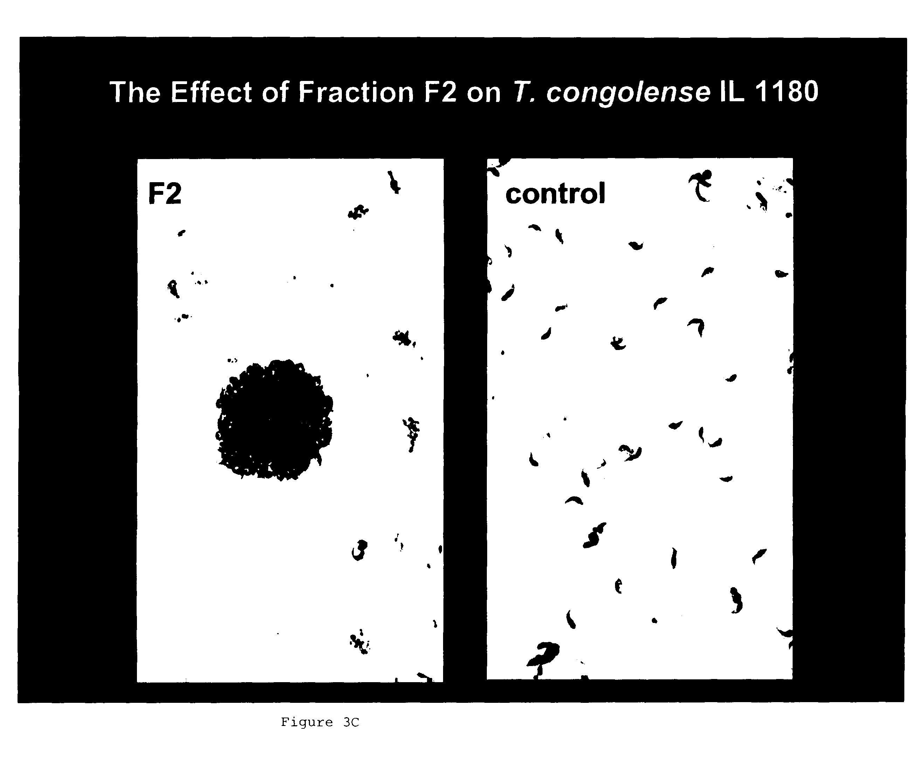

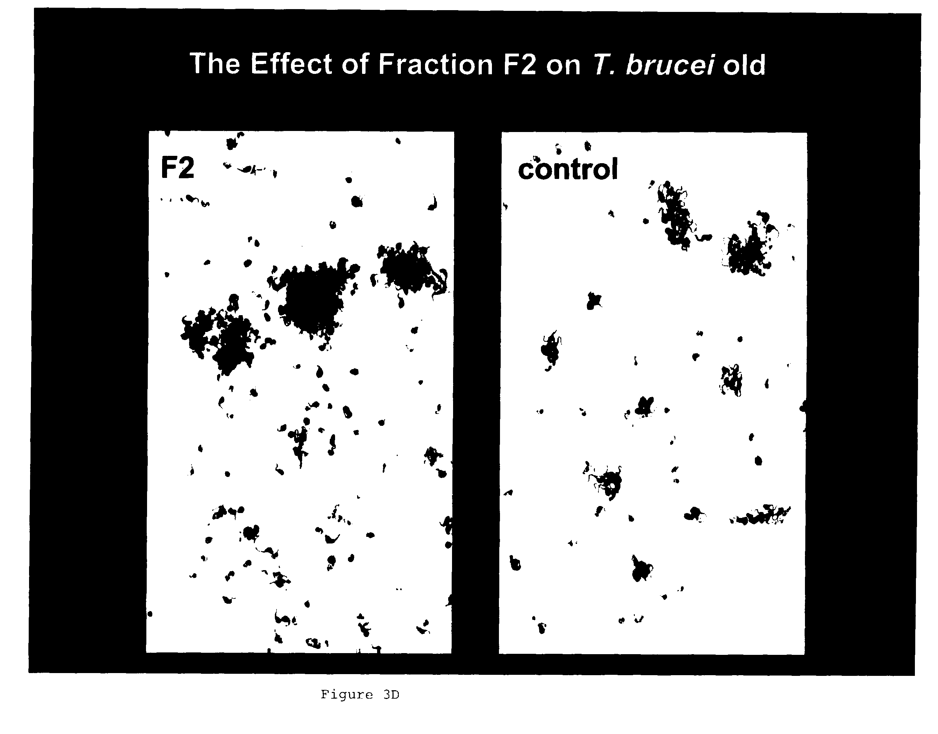

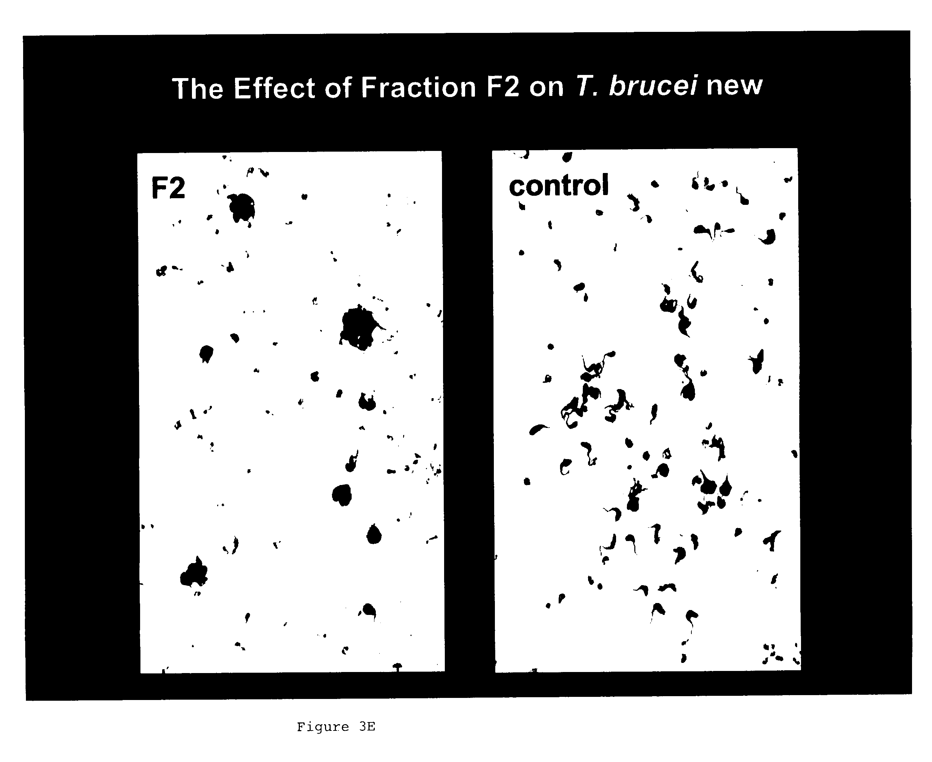

The observed effect was maximal with fresh pleomorphic cultures and diminished considerably after repeated passage of the strains. Micrographs of Giemsa stained samples from different cultures 48 hours after the addition of F2 are shown in Fig. 3. In addition to a decrease in cell number the figure shows changes in the morphology of the parasites. Many of the cells seemed to be rounding up and there was an increase in the degree of cell clumping. The sequence of events as observed under a microscope were as follows: an initial rounding of cells was followed by increased detachment from the surface of the plates and subsequent clumping. We interpreted these results as indicative for the process of apoptosis. This hypothesis was tested using the TUNEL Assay (see hereafter).

A fraction of the cells had assumed a tadpole-like structure after 48 hours and probably represents the population that has transformed to the so called Short-Stumpy (SS) form. These cells remained intact for at least another 72 hours. After this period increased lysis was observed and the total number of cells decreased rapidly (results not shown). Addition of fraction F2 to procyclic forms of *T*. *congolense, T. b. brucei* and *T. vivax* had no effect.

### DNA-endlabelling of treated and untreated trypanosomes.

DNA endlabelling of the parasite cultures 48 hours after addition of fraction F2 was done using the TUNEL Assay. The same samples were also stained by Rhodamine in order to view total DNA content and nuclear morphology. Samples were viewed by light microscopy and by electron microscopy.

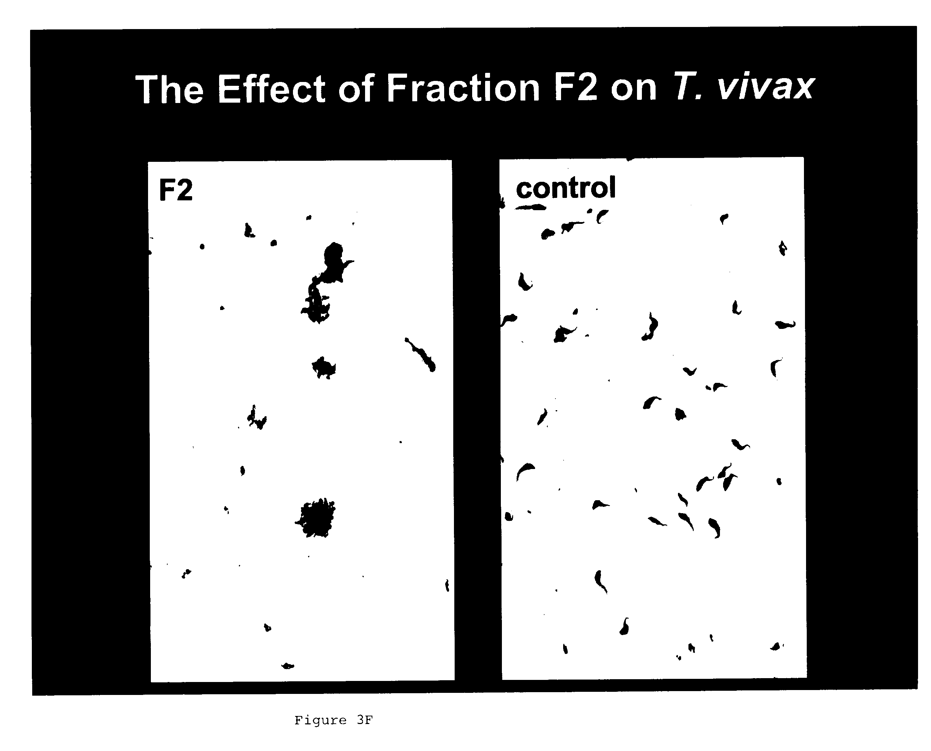

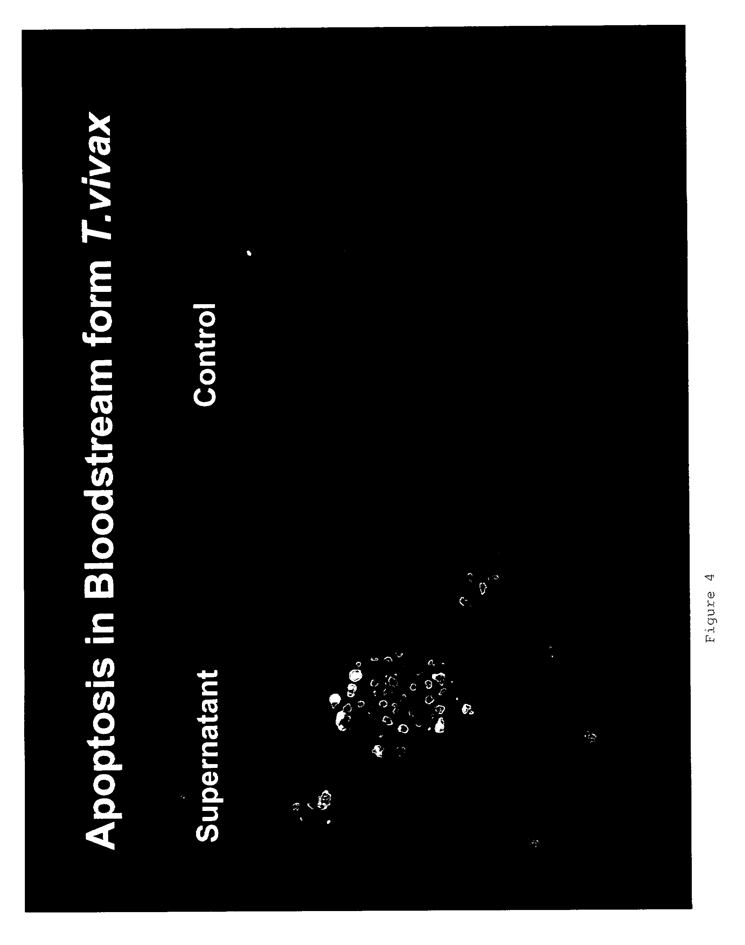

No multinucleate cells were observed. In cells that were rounded increased labelling was seen using the TUNEL assay. This effect was most pronounced with *T. vivax* (Fig. 4). The results are in favour of induced apoptosis following the addition of fraction F2.

### Differential gene expression after addition of fraction F2 to T. b. brucei.

The addition of fraction F2 to cultures of *T. b. brucei* resulted in the induction of a number of genes (results not shown). Notably the genes, which are probably involved in the process of apoptosis were seen to be up-regulated.

### RPLC purification of fraction F2

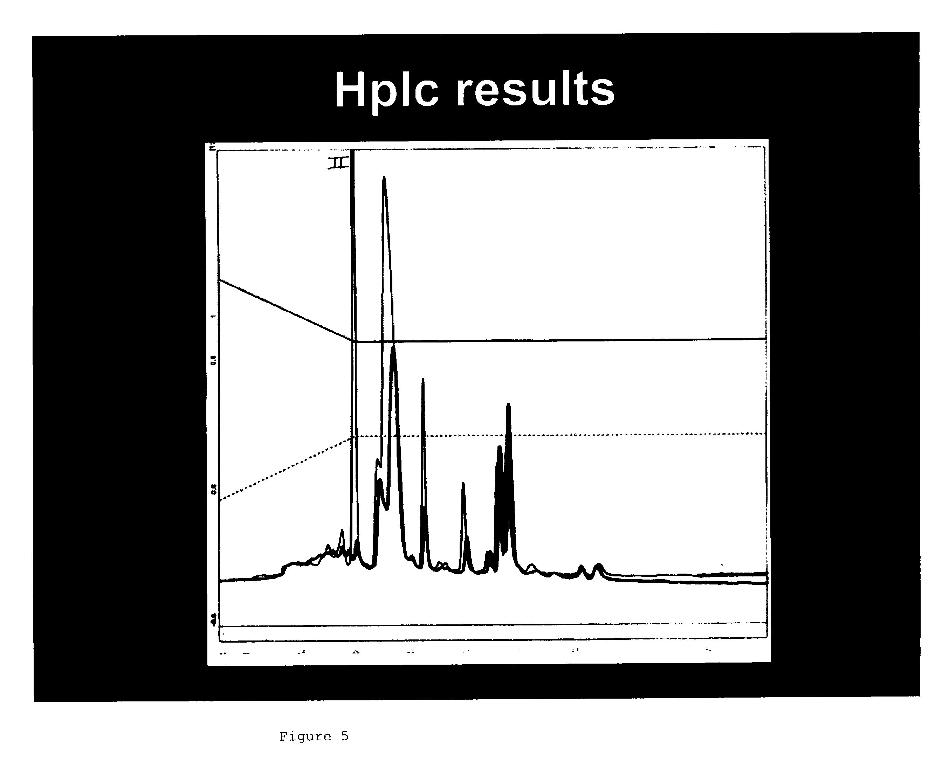

Fraction F2 was further purified using RPLC. After testing all of the collected fractions for biological activity, only the peak fractions in the range of 35-40% acetonitrile proved to be active. Most of the activity was contained in the peak labelled II (Fig. 5). This peak was absent in a sample of control medium where no parasites had grown. The absorption at 220 nm, the absorption wavelength of the peptide bond, was high while absorption at 280 nm was minimal.

Aliquots of the peak fractions stained positive with Ninhydrin spray reagent (Merck; Germany). Taken together these results would suggest that the parasites control their growth and differentiation by the excretion of small peptides. However, Thin Layer Chromatography of these fractions and of original culture supernatant gave bands that stained positive for sugar and lipid. In addition amino-acid analysis of HPLC purified active fractions showed the absence of the twenty most common amino acids.

Work is now underway to determine the chemical composition of the biologically active compounds by Mass Spectrometry and NMR.

### Cloning of ARF

ARF was identified through RADES differential display as a sequence up-regulated in actively-dividing bloodstream forms of Trypanosoma brucei brucei ILTat 1.1. Its expression in insect forms is low. Four independent clones were identified, two of 0.3 kbp and two of 0.5 kbp. From these sequences, primers were designed to get the full length sequence. A reverse primer with the mini-exon sequence was used to PCR amplify the 5' end (525 bp) and a forward primer was used with oligo (dT) to PCR amplify the 3' end (403 bp)
Primers were :
AA680517

T5166 Bloodstream form of seroderm ILTat1.1 Trypanosoma brucei brucei cDNA 5' similar to sp:P35676|ADP-Ribosylation factor [D.melanogaster], mRNA sequence.
gi|2661289|gb|AA60517. |AA680517[2661289] and AA585065
T3632 Bloodstream form of seroderme ILTat1.1
Trypanosoma brucei brucei cDNA 5' similar to gi951146|ADP-ribosylation factor [Xenopus leavis], mRNA sequence.
gi|2369618|gb|AA585065.1|AA585065[2369618].

Fragments were cloned into pGEM-T vector for sequencing. The full length of the cDNA (mini-exon to poly A tail) is 727 bp with an ORF of 543 bp encoding 182 amino acids of mol.wt. 20,580 Daltons. The nucleotide acid sequence of ARF is given in figure 6A, a predicted amino-acid sequence is given in figure 6B.

### Expression of recombinant ARF.

To express the recombinant protein primers were designed to the start and end of the protein coding region and used on genomic DNA to PCR amplify a fragment of 550 bp. The primers had a BamHI site at the 5' end and HindIII site at the 3' end. The PCR fragment was first cloned into pMOS-T, the ends sequenced to make sure everything was okay and the plasmid digested with BamHI and HindIII to release the cloned insert. This was then cloned into pQE30 cut with HindIII and BamHI. This is the expression vector with the His tag. Escherichia coli M15 cells were used. The ends were sequenced to make sure the orientation and reading frame were correct and the clone selected named pQE30/tbARF1. Cells were grown up, induced with IPTG and proteins analysed on a 12.5% SDS gel. A protein of approx. 22 kDa was detected. This was purified on a nickel column and the protein was shown to be >98% pure.

### Generation of hybridomas producing monoclonal antibodies specific for ARF.

Four Balb/C mice (10 to 12 weeks) were immunised by intraperitoneal injection of 5 µg of the recombinant protein in 100 µl PBS and 100 µl of Freunds Complete adjuvant. Two subsequent booster immunisations were undertaken with 5 µg protein in incomplete Freunds adjuvant, with each immunisation two weeks apart. Sera was tested for activity against the recombinant and native protein. Following a further 3 weeks mice with high titres were boosted once more and four days later sacrificed, spleens removed and hybridomas generated by standard methods. ELISAs on supernatants of cloned cells were undertaken using the recombinant ARF1 protein to identify antibody secreting cells that recognise ARF1. Hybridomas were recloned, checked for mAb production and 1 x 10(6) to 1 x 10(7) cells injected into the peritoneum of Balb/C mice. Ascites were recovered after 10 to 20 days.

### Blocking of anti-ARF McAb with T. congolense supernatant

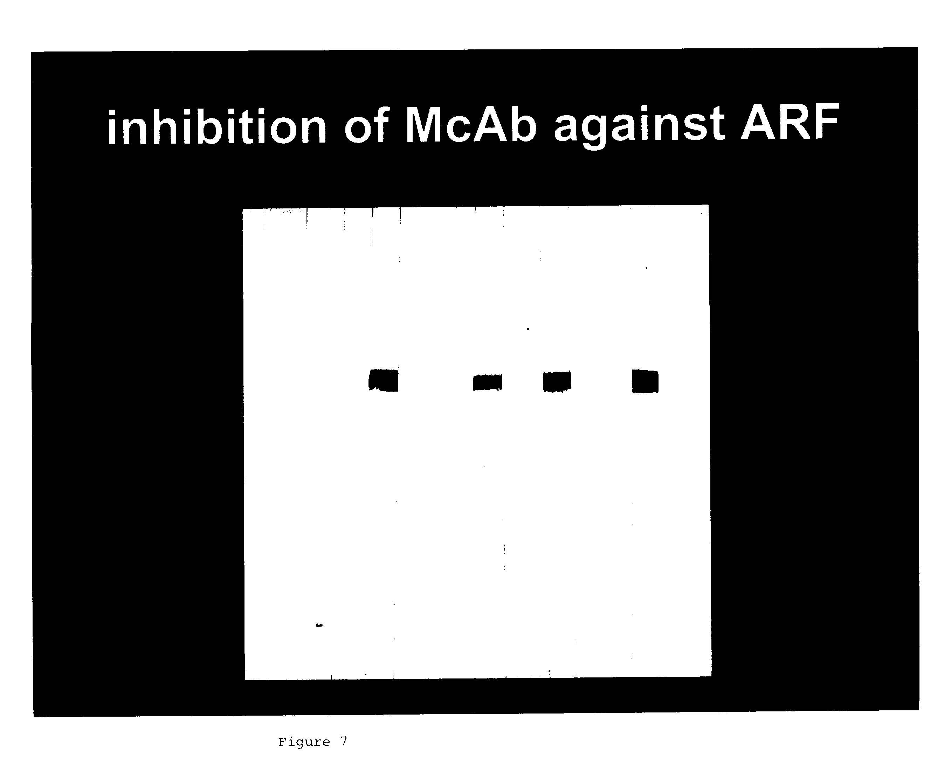

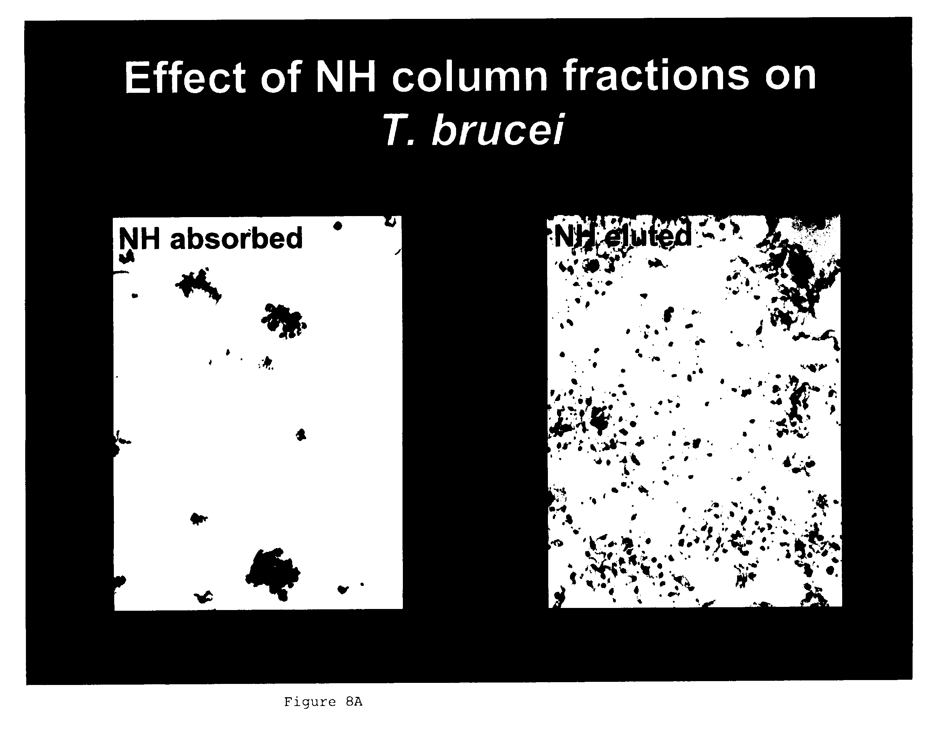

Recombinant trypanosomal ARF was run on SDS-PAGE gels and transferred to nitrocellulose paper. Strips of these blots were used for reaction with anti-ARF McAB and polyclonal anti-ARF sera in the presence and absence of supernatant fractions diluted at 1:200. As a control we used medium not containing any parasite fractions. The reaction of the McAB with ARF was blocked by the addition of supernatant fraction, polyclonal sera still reacted under these conditions (Fig. 7). Addition of control medium did not block the reaction of any of the sera with ARF. Because the activity of the supernatant disappeared after passing it over an affinity column with ARF (see next section), it seems that the McAb used and the active factor from the supernatant fraction use the same binding site on ARF.

### Activity of Sup T congo after absorption on ARF, NH and RCA affinity columns.

To investigate this possibility further, His-tagged recombinant ARF was coupled to Ni-resin (Novagen), and supernatant of T. congolense was allowed to interact with the resin. A second batch of supernatant was passed over a RCA-I column. As a control, supernatant was also passed over a column with an unrelated trypanosomal protein, nucleoside hydrolase (NH). The eluted fractions and the supernatants after passing through the respective columns were tested for biological activity in cultures of T. congolense and T. brucei.

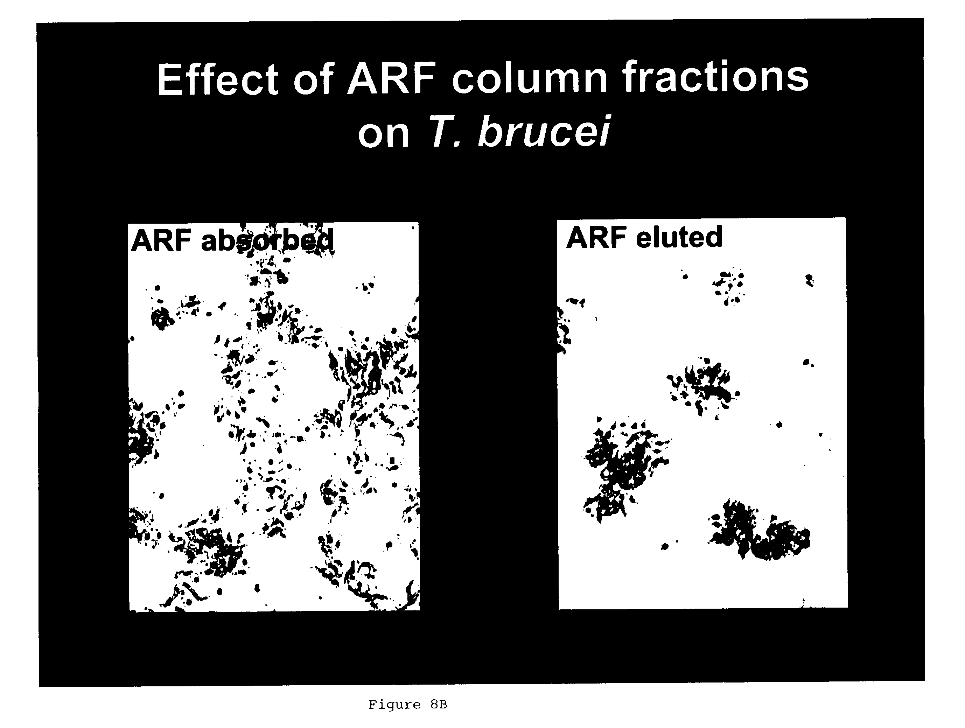

Inactivation of the supernatant occurred with the ARF and RCA-I column, while activity was retained after passing over the NH column (Fig. 8A). The eluted fraction from ARF also showed activity (Fig. 8B). Fractions eluted from RCA were much less active than the original supernatant.

### TLC of eluted fractions from ARF and RCA columns.

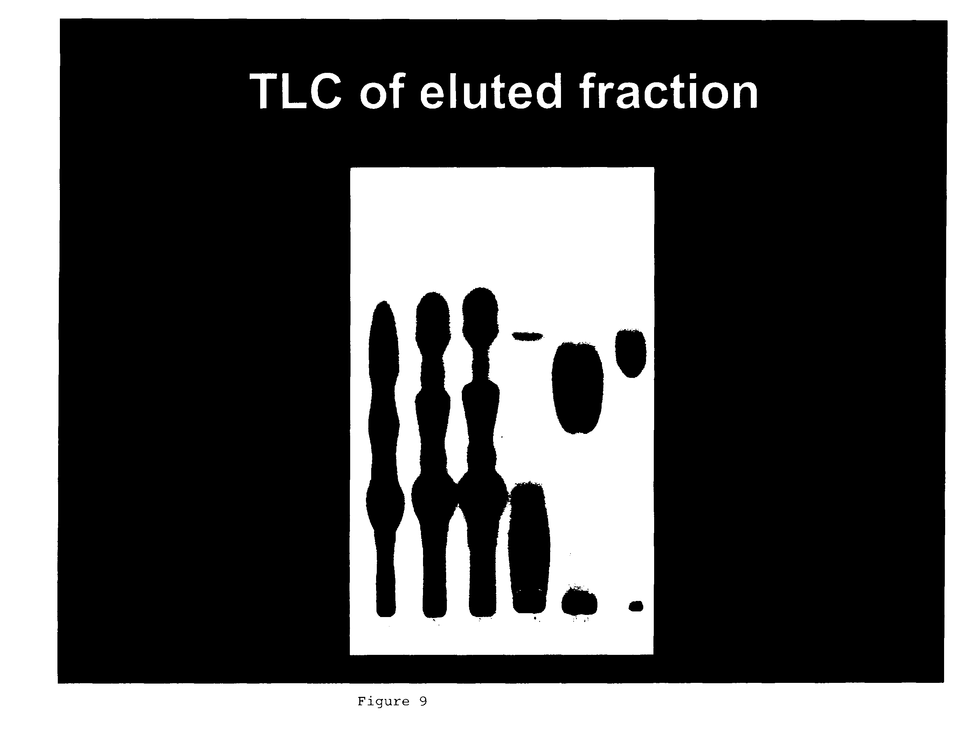

Separation of all the fractions with TLC showed two bands with similar retention times for both ARF and RCA-I eluted material (Fig. 9). Both bands contained lipid as shown by reaction with Iodine,. Sugar staining was giving very weak spots, probably because of the relatively low sensitivity of staining methods for TLC plates, but at least the RCA-I eluted material obviously contains sugar.

### Western Blot of ARF-bound lysate components from T. brucei with anti-transferrin receptor McAb.

Because ARF is reported to be involved in endocytosis, we decided to look for interactions between ARF and specific receptor proteins. This was done by passing lysates from T. brucei cultures over ARF and NH columns. The fractions that bound to these columns were eluted and separated by SDS-PAGE, proteins were transferred by Western Blotting onto nitro-cellulose and blots were incubated with a McAb against transferrin receptor from T. brucei. These experiments showed binding of transferrin receptor to ARF.

### ARF interaction with purified receptor proteins from trypanosomal Flagellar Pocket Membranes (FPM)

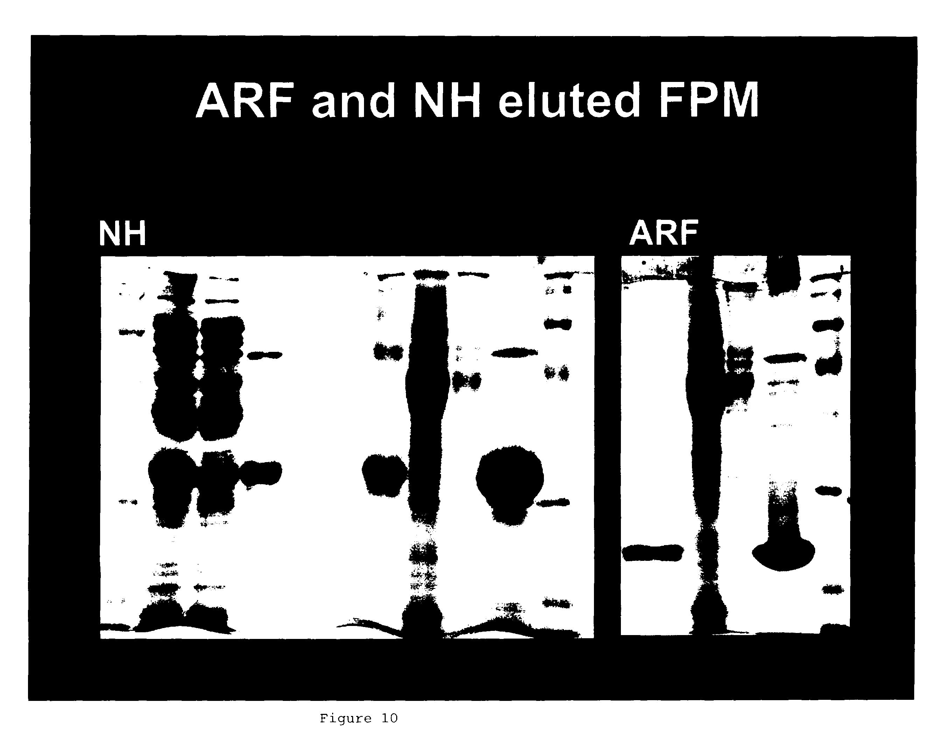

Purified trypanosomal receptors bound to an ARF affinity column and not to control column containing the unrelated protein NH (nucleoside hydrolase)(Fig. 10). Most of the receptors bound to the ARF column could be eluted by lowering the pH to 4.0. Addition of the supernatant fraction prevented the binding of the receptors. These results point to a specific interaction between ARF and receptor proteins from the trypanosomal FPM.

### Inhibition of transferrin receptor uptake by T. congo sup.

The sum of these results suggested to us that the supernatant component interacting with ARF , might also interfere with endocytosis. Therefore, cultures of T. brucei were pulse labeled with gold-labeled transferrin 48 hours after the addition of supernatant fraction. We have shown that transferrin in cells treated with supernatant was almost exclusively located at the cell surface, while in untreated cells transferrin was also found in the cytoplasm. The same samples were also probed with anti-transferrin receptor McAb. The receptor was found to co-localise with the gold-labeled transferrin.

### BRIEF DESCRIPTION OF THE DRAWINGS

- Fig. 1: Gel filtration of T. congolense 1180 conditioned medium.
Two ml of concentrated conditioned medium was separated using a 200 ml Sephadex G10 column. 7.5 ml fractions were collected. OD at 210 nm is shown as a function of fraction number.
- Fig. 2: Effect of T. congolense supernatant on T. brucei. The figure shows trypanosomes grown for 48 hours in the presence or absence of supernatant.
- Fig. 3: Effect of the addition of Fraction F2 from T. congolense supernatant on different strains of Trypanosoma (a) *T. congolense* IL 3338, (b) *T*. *congolense* IL 2642, (c) *T. congolense* IL 1180, (d) *T*. *brucei* old, (e) *T. brucei* new, (f) *T. brucei vivax.*
- Fig. 4: Apoptosis in bloodstream from *T. vivax.* Trypanosomes were grown for 48 hours or without fraction F2 and labelled using TdT endlabelling (see methods). Shown is the resulting fluorescence of cells treated this way.
- Fig. 5: HPLC results. Fraction F2 from T. congolense supernatant was analysed using HPLC (see methods). The separation profiles of this fraction is shown compared to the separation profile of control medium. H indicates the peak fraction with highest biological activity.
- Fig. 6: A) Nucleotide sequence encoding a trypanosomal ARF.
B) Predicted amino-acid sequence of a trypanosomal ARF.
- Fig. 7: Inhibition of McAb against ARF by T. congolense supernatant. Arf was separated by SDS-PAGE and the protein was transferred to nitrocellulose. The blot was cut into strips that were incubated with McAb and polyclonal serum against ARF in the presence or absence of T. congolense supernatant diluted 1:200. As a control strips were incubated with medium control fraction 1:200. Lanes: 1) McAb + sup 2) McAb + medium control 3) polyclonal serum + sup 4) polyclonal serum + medium control 5) McAb only.
- Fig. 8: Effect of NH column fractions (a) and ARF column fractions (b) on T. brucei.
T. congolense supernatant was passed over affinity columns containing bound NH or ARF. The effect of the effluent versus the eluted (bound) material is shown.
- Fig. 9: TLC of eluted fractions from ARF and RCA-1 affinity columns.
Lanes: 1) supernatant, 2) sup absorbed over ARF, 3) sup absorbed over RCA-1, 4) material eluted from ARF, 5) material eluted from RCA-1 (note the lactose used for elution from the lectin column), 6) lactose.
- Fig. 10: ARF and NH eluted FPM (flagellar pocket membrane). Purified FPM was absorbed on affinity columns containing ARF or NH and bound material was eluted by lowering the pH of the buffer. Various samples were analysed by SDS-PAGE.
Lanes:1) marker proteins 2) lysate of NH recombinant
3) same lysate after absorption on novagen column
4) purified NH protein 5) NH eluted from column
6) T. brucei lysate 7) purified FPM 8) eluate from NH column after FPM absorption 9) protein markers 10) purified ARF 11) T. brucei lysate 12) purified FPM 13) eluate from ARF column after FPM absorption 14) marker proteins.

## Claims

1. A trypanosome culture derived factor capable of at least in part inhibiting growth of a trypanosome and/or inducing cell death of said trypanosome, said factor obtainable by harvesting culture medium of a culture of trypanosomes and purifying said factor from said culture medium, wherein said factor has an apparent Molecular Weight of less than 2000 Dalton.

2. A factor according to claim 1, capable of exerting said inhibiting and/or inducing effect on a trypanosome from at least three different strains of trypanosomes, preferably of *Trypanosoma congolense, Trypanosoma brucei* and *Trypanosoma vivax.*

3. A factor according to claim 1 or claim 2, wherein said factor has an apparent Molecular Weight of less than 1000 Dalton.

4. A factor according to anyone of claims 1-3, wherein said culture is a late log phase culture of trypanosomes.

5. A factor according to anyone of claims 1-4, wherein said culture of trypanosomes is a culture of *Trypanosoma congolense* and/or *Trypanosoma brucei.*

6. A factor according to anyone of claims 1-5, capable exerting said inhibiting and/or inducing effect on a bloodstream form trypanosome.

7. A factor according to anyone of claims 1-6, wherein said factor is capable of interfering at least in part with the function of an adenosinediphosphate ribosylation factor (ARF), preferably a trypanosome associated ARF.

8. A method for obtaining a factor according to anyone of claims 1-7, comprising harvesting culture medium of a culture of trypanosomes and subjecting said culture medium to a purification procedure comprising,
- freeze drying said culture medium,
- dissolving freeze dried medium with a solution of ammonium bicarbonate (0,1 M),
- subjecting dissolved freeze dried culture medium to ultrafiltration through an Amicon YM2 filter,
- subjecting the filtrate of said ultrafiltration to a gel filtration on a sephadex G10 gelfiltration column,
- eluting said factor from said gelfiltration column with a solution of ammonium bicarbonate (0,1 M) and collecting fractions.
- freeze drying collected fractions suspected of comprising said factor,
- dissolving said freeze dried material with an aqueous solution comprising 0.1% trifluoro acetic acid,
- subjecting dissolved freeze dried material to reverse phase high pressure liquid chromatography and collecting fractions.

9. An antibody with a specificity for a factor according to anyone of claims 1-7.

10. An antibody with a specificity for a binding site of a factor according to anyone of claims 1-7 on ARF.

11. An antibody with a specificity for an ARF bound to a factor according to anyone of claims 1-7.

12. An anti-idiotypic antibody of an antibody according to anyone of claims 9-11.

13. A proteinaceous molecule capable of binding to a binding site of a factor according to anyone of claims 1-7 on ARF or a functional part, derivative and/or analogue thereof.

14. A proteinaceous molecule according to claim 13, wherein said ARF is a Trypanosome ARF.

15. An isolated and/or recombinant nucleic acid or a functional part, derivative and/or analogue thereof, encoding a trypanosome ARF, which in *Trypanosome brucei* comprises a sequence as depicted in figure 6A.

16. A trypanosome ARF or a functional part, derivative and/or analogue thereof, expressed from a nucleic acid according to claim 15.

17. An ARF according to claim 16 comprising an amino-acid sequence as depicted in figure 6B, or a functional part, derivative and/or analogue thereof.

18. A method for the treatment of an individual suffering from or at risk of suffering from Trypanosomosis comprising administering to said individual a factor according to anyone of claims 1-7, an antibody according to anyone of claims 9-12 and/or a proteinaceous molecule according to claim 13 or claim 14.

19. A method according to claim 18, wherein said individual is a human, or a life-stock animal.

20. A pharmaceutical composition comprising a factor according to anyone of claims 1-7, an antibody according to anyone of claims 9-12 and/or a proteinaceous molecule according to claim 13 or claim 14, and a pharmaceutically acceptable carrier.

21. Use of a factor according to anyone of claims 1-7, an antibody according to anyone of claims 9-12 and/or a proteinaceous molecule according to claim 13 or claim 14, as a pharmaceutical.

22. Use of a factor according to anyone of claims 1-7, an antibody according to anyone of claims 9-12 and/or a proteinaceous molecule according to claim 13 or claim 14, for the preparation of medicament for the treatment of Trypanosomosis.

23. A vaccine for preventing and/or treating at least in part Trypanosomosis comprising an antibody according to anyone of claims 9-12 and/or a proteinaceous molecule according to claim 13 or claim 14, and a suitable adjuvant.


### Annex to the application documents-subsequently filed sequences listing

SEQUENCE LISTING

<110> Stichting Dienst Landbouwkundig Onderzoek, et al

<120> Means and methods for the prevention and or treatment of
Trypanosomosis

<130> P49003EP00

<140> 99201300.3
<141> 1999-04-26

<160> 7

<170> PatentIn Ver. 2.1

<210> 1
<211> 712
<212> DNA
<213> trypanosoma

<220>
<223> Description of Combined DNA/RNA Molecule: trypanosomal ARF1

<220>
<221> CDS
<222> (76)..(621)

<220>
<223> /note="DNA and Amino-Acid sequence of trypanosomal ARF1"

<400> 1
ctatattgca aacactgtgt gtgtgtgtgg aaggaccgta accaaaagta aataaggtta 60

ataaagtttg ctgtg atg ggt caa tgg ttg gct tcc gct ttc aaa tcc ctt 111
Met Gly Gln Trp Leu Ala Ser Ala Phe Lys Ser Leu
1               5                   10

gtg gga aag cag gag gtg cgt att ctt atg gtc ggc ctt gat gct gct 159
Val Gly Lys Gln Glu Val Arg Ile Leu Met Val Gly Leu Asp Ala Ala
        15                  20                  25

gga aag acg acg atc ctg tac aaa ttg aaa ctt ggc gaa att gtg acg 207
Gly Lys Thr Thr Ile Leu Tyr Lys Leu Lys Leu Gly Glu Ile Val Thr
    30                  35                  40

acg att cct acc att ggt ttt aat gtt gag act gtg gaa tac aag aac 255
Thr Ile Pro Thr Ile Gly Phe Asn Val Glu Thr Val Glu Tyr Lys Asn
45                  50                  55                  60

ctg aaa ttc acc atg tgg gat gtt ggt ggt caa gat gtt ctt cgt ccg 303
Leu Lys Phe Thr Met Trp Asp Val Gly Gly Gln Asp Val Leu Arg Pro
                65                  70                  75

ctg tgg cgc cac tac tac caa aat acc aac ggg att atc ttt gtt gtg 351
Leu Trp Arg His Tyr Tyr Gln Asn Thr Asn Gly Ile Ile Phe Val Val
            80                  85                  90

gat tca aac gac aag gaa cgt gtg ggc aaa gcc cgc cag gag ttg gaa 399
Asp Ser Asn Asp Lys Glu Arg Val Gly Lys Ala Arg Gln Glu Leu Glu
        95                  100                 105

aag atg ctt tct gag gat gaa ttg agg aat gcc gtg ctc ctt gtt ttc 447
Lys Met Leu Ser Glu Asp Glu Leu Arg Asn Ala Val Leu Leu Val Phe
    110                 115                 120

gcc aac aag cag gac tta ccg aat gcg atg agc aca acg gaa gtc gcg     495
Ala Asn Lys Gln Asp Leu Pro Asn Ala Met Ser Thr Thr Glu Val Ala
125                 130                 135                 140

gag aaa ctt ggg ctg cag tct gtg cgc cag cgc aac tgg tat atc caa     543
Glu Lys Leu Gly Leu Gln Ser Val Arg Gln Arg Asn Trp Tyr Ile Gln
                145                 150                 155

ggt tgc tgt gct aca acg gct cag ggc ctg tat gaa ggt ctc gat tgg     591
Gly Cys Cys Ala Thr Thr Ala Gln Gly Leu Tyr Glu Gly Leu Asp Trp
            160                 165                 170

ctg tca gca aac att aag aag agt atg aaa tagtactccg ctgaaactct      641
Leu Ser Ala Asn Ile Lys Lys Ser Met Lys
        175                 180

taggttttgt tttccttgtt gtgattgttc tggtgatgtg ccctccaatt gataaaaaaa 701

aaaaaaaaaa a                                                        712


<210> 2
<211> 182
<212> PRT
<213> trypanosoma

<220>
<223> Description of Combined DNA/RNA Molecule: trypanosomal ARF1

<400> 2
Met Gly Gln Trp Leu Ala Ser Ala Phe Lys Ser Leu Val Gly Lys Gln
  1               5                  10                  15

Glu Val Arg Ile Leu Met Val Gly Leu Asp Ala Ala Gly Lys Thr Thr
             20                  25                  30

Ile Leu Tyr Lys Leu Lys Leu Gly Glu Ile Val Thr Thr Ile Pro Thr
         35                  40                  45

Ile Gly Phe Asn Val Glu Thr Val Glu Tyr Lys Asn Leu Lys Phe Thr
     50                  55                  60

Met Trp Asp Val Gly Gly Gln Asp Val Leu Arg Pro Leu Trp Arg His
 65                  70                  75                  80

Tyr Tyr Gln Asn Thr Asn Gly Ile Ile Phe Val Val Asp Ser Asn Asp
                 85                  90                  95

Lys Glu Arg Val Gly Lys Ala Arg Gln Glu Leu Glu Lys Met Leu Ser
            100                 105                 110

Glu Asp Glu Leu Arg Asn Ala Val Leu Leu Val Phe Ala Asn Lys Gln
        115                 120                 125

Asp Leu Pro Asn Ala Met Ser Thr Thr Glu Val Ala Glu Lys Leu Gly
    130                 135                 140

Leu Gln Ser Val Arg Gln Arg Asn Trp Tyr Ile Gln Gly Cys Cys Ala
145                 150                 155                 160

Thr Thr Ala Gln Gly Leu Tyr Glu Gly Leu Asp Trp Leu Ser Ala Asn
                165                 170                 175

Ile Lys Lys Ser Met Lys
            180

<210> 3
<211> 25
<212> PRT
<213> trypanosoma

<220>
<223> Description of Combined DNA/RNA Molecule: trypanosomal ARF1

<400> 3
Cys Cys Trp Lys Asp Asp Asp Pro Val Gln Ile Glu Thr Trp Arg Asn
 1               5                  10                  15

Cys Asp Asp Asp Ser Tyr His Trp Phe
            20                  25


<210> 4
<211> 58
<212> PRT
<213> trypanosoma

<220>
<223> Description of Combined DNA/RNA Molecule: trypanosomal ARF1

<400> 4
Asp Cys Gly Ile Gln Glu Pro Glu Ile His His Val Gly Cys Trp Trp
 1               5                  10                  15

Ser Arg Cys Ser Ser Ser Ala Val Ala Pro Leu Leu Pro Lys Tyr Gln
            20                  25                  30

Arg Asp Tyr Leu Cys Cys Gly Phe Lys Arg Gln Gly Thr Cys Gly Gln
        35                  40                  45

Ser Pro Pro Gly Val Gly Lys Asp Ala Phe
    50                  55


<210> 5
<211> 52
<212> PRT
<213> trypanosoma

<220>
<223> Description of Combined DNA/RNA Molecule: trypanosomal ARF1

<400> 5
Ile Glu Glu Cys Arg Ala Pro Cys Phe Arg Gln Gln Ala Gly Leu Thr
 1               5                  10                  15

Glu Cys Asp Glu His Asn Gly Ser Arg Gly Glu Thr Trp Ala Ala Val
            20                  25                  30

Cys Ala Pro Ala Gln Leu Val Tyr Pro Arg Leu Leu Cys Tyr Asn Gly
        35                  40                  45

Ser Gly Pro Val
    50


<210> 6
<211> 9
<212> PRT
<213> trypanosoma

<220>
<223> Description of Combined DNA/RNA Molecule: trypanosomal ARF1

<400> 6
Arg Ser Arg Leu Ala Val Ser Lys His
  1               5

<210> 7
<211> 4
<212> PRT
<213> trypanosoma

<220>
<223> Description of Combined DNA/RNA Molecule: trypanosomal ARF1

<400> 7
Glu Glu Tyr Glu
  1